# EUROPEAN PATENT SPECIFICATION

(11) **EP 2 888 275 B1**
(45) Date of publication and mention of the grant of the patent: **27.07.2016**
(21) Application number: 13762381.5
(22) Date of filing: 20.08.2013
(51) Int. Cl.: C07K 14/705

(54) **B-CELL ACTIVATING FACTOR FOR INCREASING MUCOSAL IMMUNITY OF INFANTS AS WELL AS SUCKLINGS AND A PREPARATION CONTAINING THIS FACTOR**
B-ZELL-AKTIVIERENDER FAKTOR ZUR ERHÖHUNG DER MUKOSALEN IMMUNITÄT VON KINDERN SOWIE SÄUGLINGEN UND ZUBEREITUNG MIT DIESEM FAKTOR
FACTEUR D'ACTIVATION DE LYMPHOCYTES B POUR AUGMENTER L'IMMUNITÉ DES MUQUEUSES D'ENFANTS EN BAS ÂGE ET DE NOURRISSONS ALLAITÉS, ET PRÉPARATION CONTENANT CE FACTEUR

(30) Priority: 22.08.2012 CZ 20120561
(43) Date of publication of application: 01.07.2015
(73) Proprietor: Masarykova Univerzita, 601 77 Brno (CZ)
(72) Inventor: BIENERTOVA VASKU, Julie, 60200 Brno (CZ)
(74) Representative: Hartvichova, Katerina
(86) International application number: PCT/CZ2013/000098
(87) International publication number: WO 2014/029374

(56) References cited:
- WO-A2-2004/081043
- TERTILT C ET AL: "Expression of B-Cell Activating Factor Enhances Protective Immunity of a Vaccine against Pseudomonas aeruginosa", INFECTION AND IMMUNITY, AMERICAN SOCIETY FOR MACROBIOLOGY, USA, vol. 77, no. 7, 1 July 2009 (2009-07-01), pages 3044-3055, XP002591578, ISSN: 0019-9567, DOI: 10.1128/IAI.00927-08 [retrieved on 2009-04-13] cited in the application
- G. A. LIED ET AL: "Functional and Clinical Aspects of the B-Cell-Activating Factor (BAFF): A Narrative Review", SCANDINAVIAN JOURNAL OF IMMUNOLOGY, vol. 73, no. 1, 5 January 2011 (2011-01-05), pages 1-7, XP55084054, ISSN: 0300-9475, DOI: 10.1111/j.1365-3083.2010.02470.x

## Description

### Field of Art

The present invention relates to the B-cell activating factor (BAFF) intended for use in increasing the immunity of infants or sucklings and to peroral preparations containing BAFF.

### Background Art

B-cells play an important role in the humoral as well as cellular immune response. Immature B-cells originate in bone marrow and migrate to spleen, where they become transitional B-cells, whereas some of them further differentiate into mature B-cells. Generally, the presence of the B-cell activating factor (BAFF) is of essential importance for normal development of B-cells.

B-cell activating factor (BAFF, also called BLyS, Tall-1, or TNFSF13), a member of the TNF (tumor necrosis factor) family, is an important cytokine that controls B-cell survival and maturation. BAFF triggers the production of specific subclasses of antibodies in the B-cells, such as IgG, IgA, and IgE, and is also involved in the immunological class-switching reactions. It seems that the aberrant signaling in the pathways that require BAFF is closely related to numerous diseases, e.g. allergies, autoimmune diseases, infections as well as some cancer diseases, in which dys-regulation occurs both at the level of humoral and cellular immunity (Cerutti A, Puga I, Cols M: Innate control of B cell responses. Trends Immunol. 2011 May; 32(5):202-11; Lied GA, Berstad A.: 48. Functional and clinical aspects of the B-cell-activating factor (BAFF): a narrative review. Scand J Immunol. 2011 Jan;73(1):1-7.; Cancro MP: The BLyS family of ligands and receptors: an archetype for niche-specific homeostatic regulation. Immunol Rev. 2004 Dec;202:237-49.).

BAFF is an important factor in numerous immunostimulatory reactions. Tertil et al. (Tertilt C, Joh J, Krause A, Chou P, Schneeweiss K, Crystal RG, Worgall S. Expression of B-cell activating factor enhances protective immunity of a vaccine against Pseudomonas aeruginosa. Infect Immun. 2009 Jul;77(7):3044-55) reported in 2009 that transient increased expression of BAFF in vivo was capable of enhancing the antigen-specific humoral immunity a thus may be extremely useful in vaccines intended for induction of antibodies against extracellular bacteria. The authors pointed out that the short-term increase of BAFF lead to enhanced immune reaction against *Pseudomonas aeruginosa* when vaccinating with inactivated bacteria and also propose that BAFF could be used as an efficient adjuvant in the vaccines, due to its immunostimulatory effects.

It is well known that mucosal immunity in gastrointestinal tract of infants (children of suckling age) or sucklings (offspring of mammals of suckling age) fed by artificial nutrition is compromised compared to that of breastfed infants or sucklings, which causes a significantly higher incidence of infections of the gastrointestinal tract in the non-breastfed infants or sucklings.

### Disclosure of the Invention

The present invention relates to the B-cell activating factor (BAFF) for use in a method of increasing mucosal immunity in the gastrointestinal tract of infants or sucklings.

The infants are children in the suckling age, which corresponds to the age range from 0 to 2 years.
The sucklings are offspring of mammals in the suckling age.

Within the framework of the present invention, it was surprisingly found that BAFF is secreted in significant amounts into breast milk, in particular in humans. The levels of BAFF in the breast milk reach extreme values shortly after the delivery, 25-times exceeding the circulating values in the serum of the mother, and these levels show continuous exponential decrease during the breastfeeding period. The presence of BAFF in colostrum provides a strong maturation signal for B-cells in the early postnatal period, while the importance of this signal decreases with the time elapsed from the delivery along with the increase of the specific immunity of the offspring. BAFF participates in physiological class-switching of IgG, IgA, and IgE antibody subtypes, which is a mechanism by which the locally produced antibodies are modified, and therefore releasing of BAFF into breast milk affects B-cells in the gastrointestinal tract mucosal lymphoid tissue (GALT) of the newborns, which provides the infant with an important protection against infections from external environment. BAFF causes accumulation and maturation of B-cells in the newborn as well as increased intestinal production of IgA in GALT.

A further subject of the present invention is a preparation for peroral administration, destined for stimulation of mucosal immunity in the gastrointestinal tract of infants or sucklings, containing BAFF. In a preferred embodiment, it is an infant milk formula or suckling milk formula containing BAFF or a suspension of BAFF in water.

The preparation for increasing the mucosal immunity is intended for frequent artificial infant or suckling nutrition, i.e., in the age range between 0 and 2 years in infants. In a preferred embodiment, the preparation is intended for the infants at the age between 0 and 6 months.

The preparation contains 200 to 3500 pg of BAFF/ml of the preparation, more preferably 400 to 2500 pg/ml, most preferably 500 to 2000 pg/ml.

The infant milk formula is a product on the basis of cow milk or milk of other animals and/or other components that have been approved as suitable for nutrition of infants.
The infant milk formula prepared for final consumption in line with the manufacturer's instructions should have the minimum energetic value of 250 kJ per 100 ml, while the energetic value should not exceed 295 kJ per 100 ml, and it should contain per each 100 kcal the minimum to maximum levels of the nutrients defined in Table 1 (Koletzko B,

Baker S, Cleghorn G, Neto UF, Gopalan S, Hernell O, Hock QS, Jirapinyo P, Lonnerdal B, Pencharz P, Pzyrembel H, Ramirez-Mayans J, Shamir R, Turck D, Yamashiro Y, Zong-Yi D.: Global standard for the composition of infant formula: recommendations of an ESPGHAN coordinated international expert group. J Pediatr Gastroenterol Nutr. 2005 Nov;41(5):584-99).

Codex Alimentarius of the Food and Agriculture Organization (FAO) of the United Nations in cooperation with the World Health Organization (WHO) develops in the long term the standards for foodstuff and prepares related texts so that the health of the consumer is protected and the adequate production processes is globally ensured. The Standard of the Codex Standard on Infant Formula was adopted in 1981 based on scientific knowledge available in the 1970s and is currently being revised. As part of this process, the Codex Committee on Nutrition and Foods for Special Dietary Uses asked the ESPGHAN Committee
on Nutrition to initiate a consultation process with the international scientific community to provide a proposal on nutrient levels in infant formulae, based on scientific analysis and taking into account existing scientific reports on the subject. The ESPGHAN Committee has therefore created an international expert group that adopted in 2005 new recommendations concerning the composition of the infant formula (Table 1).

The composition of the infant formulas are thus well known to those skilled in the art.

**Table 1: Recommended standards for preparation of infant formulas according to ESPGHAN**

| Component | Unit | Minimum | Maximum |
|---|---|---|---|
| Energy | kcal/100 ml | 60 | 70 |
| Cow milk protein | g/100 kcal | 1,8 | 3 |
| Total fat | g/100 kcal | 4,4 | 6,0 |
| Linoleic acid | g/100 kcal | 0,3 | 1,2 |
| Alpha-linolenic acid | mg/100 kcal | 50 | - |
| Ratio linoleic acid / alpha-linolenic acid | | 5:1 | 15:1 |
| Lauric and myristic acid | % in fat | NS | 20 |
| Trans fatty acids | % in fat | NS | 3 |
| Erucic acid | % in fat | NS | 1 |
| Total carbohydrates | g/100 kcal | 9 | 14 |
| Vitamin A | µg RE/100 kcal | 60 | 180 |
| Vitamin D3 | µg / 100 kcal | 1 | 2,5 |
| Vitamin E | mg alpha-TE/ 100 kcal | 0,5 | 5 |
| Vitamin K | µg / 100 kcal | 4 | 25 |
| Thiamin | µg / 100 kcal | 60 | 300 |
| Riboflavin | µg / 100 kcal | 80 | 400 |
| Niacin | µg / 100 kcal | 300 | 1500 |
| Vitamin B6 | µg / 100 kcal | 35 | 175 |
| Vitamin B12 | µg / 100 kcal | 0,1 | 0,5 |
| Folic acid | µg / 100 kcal | 400 | 2000 |
| Pantothenic acid | µg / 100 kcal | 10 | 50 |
| Vitamin C | mg / 100 kcal | 10 | 30 |
| Biotin | µg / 100 kcal | 1,5 | 7,5 |
| Iron | mg / 100 kcal | 0,3 | 1,3 |
| Calcium | mg / 100 kcal | 50 | 140 |
| Phosphorus | mg / 100 kcal | 25 | 90 |
| Ratio calcium / phosphorus | mg / mg | 1:1 | 2:1 |
| Magnesium | mg / 100 kcal | 5 | 15 |
| Sodium | mg / 100 kcal | 20 | 60 |
| Chloride | mg / 100 kcal | 50 | 160 |
| Potassium | mg / 100 kcal | 60 | 160 |
| Manganese | µg / 100 kcal | 1 | 50 |
| Fluoride | µg / 100 kcal | NS | 60 |
| Iodine | µg / 100 kcal | 10 | 50 |
| Selenium | µg / 100 kcal | 1 | 9 |
| Copper | µg / 100 kcal | 35 | 80 |
| Zinc | mg / 100 kcal | 0,5 | 1,5 |
| Choline | mg / 100 kcal | 7 | 50 |
| Myo-inositol | mg / 100 kcal | 4 | 40 |
| L-carnitine | mg / 100 kcal | 1,2 | NS |

| | | | |
|---|---|---|---|
| Note: NS = Not Specified | | | |

Given there is a tendency in manufacturing the infant formulas towards the maximum similarity with the breast milk, it is appropriate and logical that the levels of BAFF in the infant formula should mimic the dynamics of the levels in the human breast milk: therefore it is particularly recommended that the preparations according to the present invention, if these are infant milk formulas, contain 1500 to 3500 pg BAFF / ml of milk, preferably 2000 pg/ml for the children at the age of 0 to 3 months, and 400 to 800 pg/ml, preferably 500 pg/ml, for the children at the age of 3 to 6 months.

Particularly, the preparations according to the present invention are suitable for stimulation of mucosal immunity in premature newborns and non-breastfed newborns as well as in older infants on infant milk formula. The preparations are of particular importance for infants aged 0 to 6 months, as therein contained BAFF may be very important for development of the pool of memory B-cells and for formation of the physiological microbiome.

The advantage of fortification of the infant milk formulas by BAFF is fast initiation of formation of the adequate mucosal immunity of the child, establishment of adequate microbiome as well as quick development of tolerance to individual components of the used formula within the gastrointestinal tract.

The present invention is useful for human medicine and nutrition as well as for veterinary medicine and nutrition.

### Brief Description of Figures

Figure 1 shows the contents of BAFF in the blood serum of the breastfeeding mothers in relation to the time elapsed from the delivery.
Figure 2 shows the contents of BAFF in the breast milk of the breastfeeding mothers in relation to the time elapsed from the delivery.

### Examples of carrying out the invention

### Example 1

Samples of breast milk and peripheral venous blood were acquired from seven breastfeeding women, whereas these women had spontaneous, non-complicated, physiological pregnancy, and the size of the newborns was adequate to the gestational age (AGA). The samples were taken always in pairs: breast milk - venous blood of the mother; this was done at the time of delivery (if the milk was already present), on days 1 - 3, 12 - 14, 28 - 30, 88 - 90, and 178 - 180 after the delivery, while the children were only fully breastfed during the entire duration of the study. The criteria for eligibility for the study contained: A) spontaneous conception, B) non-complicated single gravidity, C) spontaneous non-complicated delivery, D) normal results of peroral glucose tolerance test (oGTT) between the weeks 24 - 28 of the gravidity based on the WHO definition, and E) the size of the fetus adequate to the gestational age (AGA). The study has been approved by the Ethical committee of the Faculty of Medicine of Masaryk University in Brno in accordance with the Helsinki Declaration. The milk was taken by manual extraction from both breasts in the given time points of the study, while, if it was possible, 5 ml of milk was taken (especially at the time point 1 and 2, as much colostrum as possible was taken). Concentration of the BAFF protein in the maternal serum and breast milk were determined using the test set BAFF ELISA (R&D Systems, Inc. Minneapolis, USA) according to the manufacturer's instructions. Defrosted samples of milk were first delipidated by centrifugation at 12000 x g for 15 minutes at 0 °C and then diluted up to 40-times by calibration solvent in duplicates for the testing range (62.5 to 4000 pg/ml). Coefficients of variation between the tests and within the test (CV) were for BAFF in the breast milk < 6.0 % and in the maternal serum < 9.0 %, with the detection limit of 3.38 pg/ml.

Analysis of the BAFF protein in the breast milk for the entire group of women under study during the entire period did not show significant variations in the concentration of BAFF between the monitored time points [F(5;38) = 3.09, p = 0.68], however, the concentration in the breast milk dramatically changed during the duration of the study [F(5;42) = 33.08, p = 0.0000001]. We observed consistent presence of BAFF in the breast milk, while the average concentration of BAFF in the milk was at the time of delivery approximately 25-times higher than in the serum [average ± SD: 32706 ± 26172 pg/ml], 6-times higher on day 1 - 3 [average ± SD: 8769 ± 2481 pg/ml], and approximately the same in the milk and in the maternal serum on day 12 - 14 [average ± SD: 1493 ± 892 pg/ml], subsequently the levels of BAFF in the milk dropped under the level observed in the peripheral blood of the mother [average ± SD: 1091 ± 526 pg/ml] on day 28 - 30, [average ± SD: 445 ± 245 pg/ml] on day 88 - 90, and [average ± SD: 354 ± 335 pg/ml] on day 178 - 180.

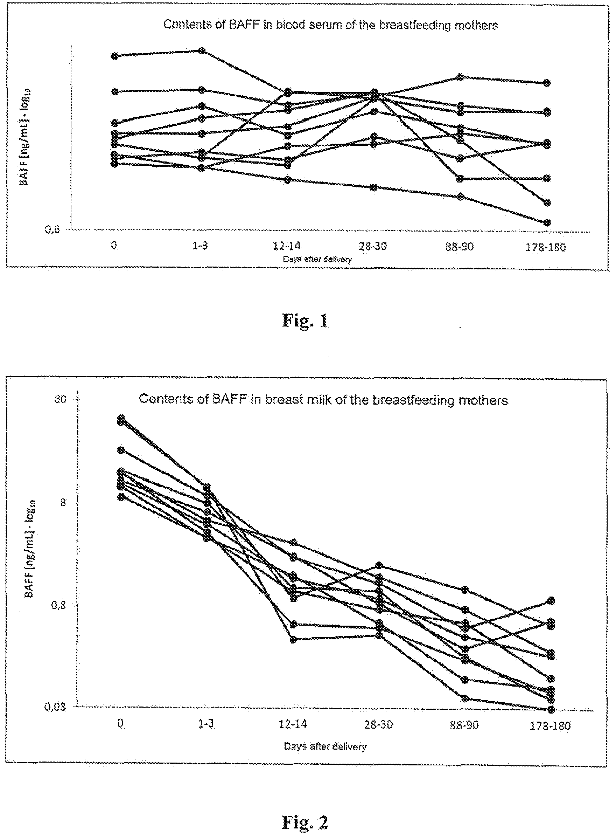

The charts enclosed show the changes of the content of BAFF in the blood serum (Figure 1) and in the breast milk (Figure 2) during the period under monitoring.

### Example 2

Composition of the preparation based on the infant milk formula for early artificial nutrition (until the end of the second month after the delivery). The infant milk formula is prepared using the preparation methods well known to those skilled in the art.

| Component | Unit | Minimum contents of the component | Maximum content of the component |
|---|---|---|---|
| Energy | kcal / 100 ml | 60 | 70 |
| Cow milk protein | g / 100 kcal | 1,8 | 3 |
| Total fat | g / 100 kcal | 4,4 | 6,0 |
| Linoleic acid | g / 100 kcal | 0,3 | 1,2 |
| Alpha-linolenic acid | mg / 100 kcal | 50 | |
| Ratio linoleic acid / alpha-linolenic acid | | 5:1 | 15:1 |
| Lauric and myristic acid | % in fat | NS | 20 |
| Trans fatty acids | % in fat | NS | 3 |
| Erucic acid | % in fat | NS | 1 |
| Total carbohydrates | g / 100 kcal | 9 | 14 |
| Vitamin A | µg RE / 100 kcal | 60 | 180 |
| Vitamin D3 | µg / 100 kcal | 1 | 2,5 |
| Vitamin E | mg alpha-TE / 100 kcal | 0,5 | 5 |
| Vitamin K | µg / 100 kcal | 4 | 25 |
| Thiamin | µg / 100 kcal | 60 | 300 |
| Riboflavin | µg / 100 kcal | 80 | 400 |
| Niacin | µg / 100 kcal | 300 | 1500 |
| Vitamin B6 | µg / 100 kcal | 35 | 175 |
| Vitamin B12 | µg / 100 kcal | 0,1 | 0,5 |
| Folic acid | µg / 100 kcal | 400 | 2000 |
| Pantothenic acid | µg / 100 kcal | 10 | 50 |
| Vitamin C | mg / 100 kcal | 10 | 30 |
| Biotin | µg / 100 kcal | 1,5 | 7,5 |
| Iron | mg / 100 kcal | 0,3 | 1,3 |
| Calcium | mg / 100 kcal | 50 | 140 |
| Phosphorus | mg / 100 kcal | 25 | 90 |
| Ratio calcium / phosphorus | mg/mg | 1:1 | 2:1 |
| Magnesium | mg / 100 kcal | 5 | 15 |
| Sodium | mg / 100 kcal | 20 | 60 |
| Chloride | mg / 100 kcal | 50 | 160 |
| Potassium | mg / 100 kcal | 60 | 160 |
| Manganese | µg / 100 kcal | 1 | 50 |
| Fluoride | µg / 100 kcal | NS | 60 |
| Iodine | µg / 100 kcal | 10 | 50 |
| Selenium | µg / 100 kcal | 1 | 9 |
| Copper | µg / 100 kcal | 35 | 80 |
| Zinc | mg / 100 kcal | 0,5 | 1,5 |
| Choline | mg / 100 kcal | 7 | 50 |
| Myo-inositol | mg / 100 kcal | 4 | 40 |
| L-carnitine | mg / 100 kcal | 1,2 | NS |
| BAFF | pg / ml of ready milk | 2000 | 3500 |

### Example 3

Composition of the preparation based on the infant milk formula for continued artificial nutrition (from the end of the second month after the delivery). The infant milk formula is prepared using the preparation methods well known to those skilled in the art.

| Component | Unit | Minimum contents of the component | Maximum content of the component |
|---|---|---|---|
| Energy | kcal / 100 ml | 60 | 70 |
| Cow milk protein | g / 100 kcal | 1,8 | 3 |
| Total fat | g / 100 kcal | 4,4 | 6,0 |
| Linoleic acid | g / 100 kcal | 0,3 | 1,2 |
| Alpha-linolenic acid | mg / 100 kcal | 50 | - |
| Ratio linoleic acid / alpha-linolenic acid | | 5:1 | 15:1 |
| Lauric and myristic acid | % in fat | NS | 20 |
| Trans fatty acids | % in fat | NS | 3 |
| Erucic acid | % in fat | NS | 1 |
| Total carbohydrates | g / 100 kcal | 9 | 14 |
| Vitamin A | µg RE / 100 kcal | 60 | 180 |
| Vitamin D3 | µg / 100 kcal | 1 | 2,5 |
| Vitamin E | mg alpha-TE / 100 kcal | 0,5 | 5 |
| Vitamin K | µg / 100 kcal | 4 | 25 |
| Thiamin | µg / 100 kcal | 60 | 300 |
| Riboflavin | µg / 100 kcal | 80 | 400 |
| Niacin | µg / 100 kcal | 300 | 1500 |
| Vitamin B6 | µg / 100 kcal | 35 | 175 |
| Vitamin B12 | µg / 100 kcal | 0,1 | 0,5 |
| Folic acid | µg / 100 kcal | 400 | 2000 |
| Pantothenic acid | µg / 100 kcal | 10 | 50 |
| Vitamin C | mg / 100 kcal | 10 | 30 |
| Biotin | µg / 100 kcal | 1,5 | 7,5 |
| Iron | mg / 100 kcal | 0,3 | 1,3 |
| Calcium | mg / 100 kcal | 50 | 140 |
| Phosphorus | mg / 100 kcal | 25 | 90 |
| Ratio calcium / phosphorus | mg/mg | 1:1 | 2:1 |
| Magnesium | mg / 100 kcal | 5 | 15 |
| Sodium | mg / 100 kcal | 20 | 60 |
| Chloride | mg / 100 kcal | 50 | 160 |
| Potassium | mg / 100 kcal | 60 | 160 |
| Manganese | µg / 100 kcal | 1 | 50 |
| Fluoride | µg / 100 kcal | NS | 60 |
| Iodine | µg / 100 kcal | 10 | 50 |
| Selenium | µg / 100 kcal | 1 | 9 |
| Copper | µg / 100 kcal | 35 | 80 |
| Zinc | mg / 100 kcal | 0,5 | 1,5 |
| Choline | mg / 100 kcal | 7 | 50 |
| Myo-inositol | mg / 100 kcal | 4 | 40 |
| L-carnitine | mg / 100 kcal | 1,2 | NS |
| BAFF | pg / ml of ready milk | 500 | 800 |

## Claims

1. B-cell activating factor for use in a method of increasing mucosal immunity in the gastrointestinal tract of infants or sucklings **characterized in that** it is administered perorally in a preparation comprising 200 to 3500 pg of B-cell activating factor /ml of the preparation.

2. A preparation comprising 200 to 3500 pg of B-cell activating factor /ml of the preparation for use in a method of increasing mucosal immunity in the gastrointestinal tract of infants or sucklings, wherein the preparation is administered perorally.

3. The preparation for use according to claim 2, wherein the preparation contains 400 to 2500 pg of B-cell activating factor /ml of the preparation.

4. The preparation for use according to claim 2, wherein the preparation contains 500 to 2000 pg of B-cell activating factor Iml of the preparation.

5. The preparation for use according to claim 2, wherein the preparation is infant milk formula or suckling milk formula containing B-cell activating factor.

6. The preparation for use according to claim 2, wherein the preparation is a suspension of B-cell activating factor in water.

## Patentansprüche

1. B-Zell-aktivierender Faktor zur Verwendung bei der Methode zur Erhöhung der Schleimhautimmunität im gastrointestinalen Trakt der Säuglinge oder der gestillten Jungtiere **dadurch gekennzeichnet, dass** er peroral in einem Präparat enthaltend 200 bis 3500 pg B-Zellaktivierenden Faktor / ml Präparat verabreicht wird.

2. Präparat enthaltend 200 bis 3500 pg B-Zell-aktivierenden Faktor / ml Präparat zur Verwendung bei der Methode zur Erhöhung der Schleimhautimmunität im gastrointestinalen Trakt der Säuglinge oder der gestillten Jungtiere, wobei dieses Präparat peroral verabreicht wird.

3. Präparat zur Verwendung nach dem Anspruch 2, wobei das Präparat 400 bis 2500 pg B-Zell-aktivierenden Faktor / ml Präparat enthält.

4. Präparat zur Verwendung nach dem Anspruch 2, wobei das Präparat 500 bis 2000 pg B-Zell-aktivierenden Faktor / ml Präparat enthält.

5. Präparat zur Verwendung nach dem Anspruch 2, wobei das Präparat Milch zur Säugling-Ersatznahrung oder Milch zur Jungtier-Ersatznahrung enthaltend den B-Zell-aktivierenden Faktor ist.

6. Präparat zur Verwendung nach dem Anspruch 2, wobei das Präparat eine Suspension des B-Zell-aktivierenden Faktors im Wasser ist.

## Revendications

1. Facteur d'activation de lymphocytes B pour utilisation dans une méthode visant à augmenter l'immunité des muqueuses dans l'appareil digestif d'enfants en bas âges ou de nourrissons allaités, **caractérisé en ce qu'**il est administré par voie orale dans une préparation contenant 200 à 3500 pg de facteur d'activation de lymphocytes B /ml de la préparation.

2. Préparation contenant 200 à 3500 pg de facteur d'activation de lymphocytes B /ml de la préparation pour utilisation dans une méthode visant à augmenter l'immunité des muqueuses dans l'appareil digestif d'enfants en bas âges ou de nourrissons allaités, la préparation étant administrée par voie orale.

3. Préparation pour utilisation selon la revendication 2, **caractérisée en ce que** la préparation contient 400 à 2500 pg de facteur d'activation de lymphocytes B /ml de la préparation.

4. Préparation pour utilisation selon la revendication 2, **caractérisée en ce que** la préparation contient 500 à 2000 pg de facteur d'activation de lymphocytes B /ml de la préparation.

5. Préparation pour utilisation selon la revendication 2, **caractérisée en ce que** la préparation est le lait de substitution pour l'alimentation d'enfants en bas âges ou le lait de substitution pour l'alimentation des nourrissons contenant le facteur d'activation de lymphocytes B.

6. Préparation pour utilisation selon la revendication 2, **caractérisée en ce que** la préparation est une suspension du facteur d'activation de lymphocytes B dans l'eau.
